(19) Europäisches Patentamt
European Patent Office
Office européen des brevets

(11) EP 0 964 740 B1

(12) EUROPÄISCHE PATENTSCHRIFT

(45) Veröffentlichungstag und Bekanntmachung des Hinweises auf die Patenterteilung:
**08.12.2004 Patentblatt 2004/50**

(21) Anmeldenummer: **98912436.7**

(22) Anmeldetag: **04.03.1998**

(51) Int Cl.[7]: **B01F 17/00**, C11D 3/37, C08G 73/06, C08G 69/10

(86) Internationale Anmeldenummer:
**PCT/EP1998/001195**

(87) Internationale Veröffentlichungsnummer:
**WO 1998/039090 (11.09.1998 Gazette 1998/36)**

(54) **Biotechnologisches Verfahren zur Herstellung von Asparaginsäure Homo- und Copolymeren**

Biotechnical process for the preparation of aspartic acid homo- or copolymers

Procédé biotechnologique pour la préparation de homo- ou copolymères d'acide aspartique

(84) Benannte Vertragsstaaten:
**AT BE CH DE DK ES FR GB IT LI NL**

(30) Priorität: **06.03.1997 DE 19709024**

(43) Veröffentlichungstag der Anmeldung:
**22.12.1999 Patentblatt 1999/51**

(73) Patentinhaber: **Bayer Chemicals AG**
**51368 Leverkusen (DE)**

(72) Erfinder:
- **JOENTGEN, Winfried**
  **D-51067 Köln (DE)**
- **GROTH, Torsten**
  **D-51519 Odenthal (DE)**
- **STEINBÜCHEL, Alexander**
  **D-48341 Altenberge (DE)**
- **HAI, Tran**
  **D-48161 Münster (DE)**
- **OPPERMANN, Fred, Bernd**
  **D-48159 Münster (DE)**

(56) Entgegenhaltungen:
**EP-A- 0 256 366** **EP-A- 0 604 813**

- **CHEMICAL ABSTRACTS, vol. 84, no. 11, 15.März 1976 Columbus, Ohio, US; abstract no. 70582, R. SIMON ET AL.: "Determination of the structure of the novel polypeptide containing aspartic acid and arginine which is found in cyanobacteria" XP002071269 & BIOCHIM. BIOPHYS. ACTA, Bd. 420, Nr. 1, 1976, Seiten 165-176,**
- **CHEMICAL ABSTRACTS, vol. 97, no. 23, 6.Dezember 1982 Columbus, Ohio, US; abstract no. 195668, N. LAWRY ET AL.: "The normal and induced occurence of cyanophycin inclusion bodies in several blue-green algae" XP002071270 & J. PHYCOL., Bd. 18, Nr. 3, 1982, Seiten 391-399,**
- **CHEMICAL ABSTRACTS, vol. 92, no. 21, 26.Mai 1980 Columbus, Ohio, US; abstract no. 177182, M. ALLEN ET AL.: "Cyanophycin granule polypeptide formation and degradation in the cyanobacterium Aphanocapsa 6308" XP002071271 & J. BACTERIOL., Bd. 141, Nr. 2, 1980, Seiten 687-693,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung bezieht sich auf die Herstellung von Polyasparaginsäure Homo- und Copolymeren durch biotechnologische Prozesse.

Kristallisations- und Agglomerationsvorgänge gehören als biologische Mineralisation zu den grundlegenden Prozessen in der belebten Natur. So sind sie beispielsweise am Skelett- oder Schalenaufbau in lebenden Organismen beteiligt. In der Natur werden diese Mineralisationsvorgänge durch natürlich vorkommende Proteine und Polysaccharide reguliert. (S. Weiner, Biochem. 22, (1983), 4139 -45; C. S. Sikes, A. P. Wheeler, in Chemical Aspects of Regulation of Mineralisation., Eds. C. S. Sikes , A. P. Wheeler University of South Alabama Publ. Services (1988), 15 - 20).

Unglücklicherweise treten sowohl in der Natur als auch im industriellen Bereich auch nicht erwünschte Mineralisationsvorgänge auf und führen zu hartnäckigen störenden Niederschlägen und Verkrustungen wie beispielsweise Zahn Plaque, Organ Steinen oder im technischen Bereich Verkrustungen an Wärmetauscheroberflächen oder Kühltürmen Partikelagglomerationen in Pigmentdispersionen, Verkrustungen auf harten (z.B. Glas Metall) und weichen (Textil) Oberflächen. In der Vergangenheit wurden verschiedene Vorschläge zur Nutzung dieses Naturwirkprinzips für technische Problemstellungen gemacht. So beschreiben die Patente US 4 534 881, US 4 585 560, US 4 587 021 die Inhibierung von Calciumcarbonat-Niederschlägen durch Proteinfraktionen, Polysaccharidfraktionen oder Polyaminosäurefraktionen aus Calciumcarbonat bildenden Organismen wie Crustaceen etc..

Weiterhin wird in der Literatur die Inhibierung mineralischer Niederschläge durch polyanionische hydrophobe Polypeptide mit Block-Copolymer Struktur und verwandte phosphorylierte Polypeptide (US 4 868 287) beansprucht. Die verwendeten Polypeptide werden durch peptidchemische Methoden hergestellt. Gemäß WO 92/17194 soll eine verbesserte Synthese dieser Polypeptide bereitgestellt werden.

Da die oben beschriebenen Proteine ihren polyanionischen Charakter durch einen hohen Asparaginsäuregehalt erhalten, werden auch Asparaginsäure Homo- und Copolymere für diesen Zweck beansprucht. Diese Polyasparaginsäuren werden jedoch alle durch chemische Synthese erhalten. So kann man beispielsweise durch thermische Polykondensation von Asparaginsäure zu Polysuccinimid und anschließende basische Hydrolyse ein Polyasparaginsäurenatriumsalz herstellen. (Kovacs et al. J. Org. Chem, 26 (1961) 1084 -1091). In weiteren Anmeldungen wird die Herstellung und Verwendung von Polyasparaginsäuren durch thermische Polykondensation von Asparaginsäure in Gegenwart saurer Katalysatoren wie Phosphorsäure beansprucht. Außerdem werden noch Polyasparaginsäuren hergestellt durch thermische Polymerisation ausgehend von Asparaginsäurevorstufen wie Maleinsäureammoniumsalz (EP 0 256 366), Maleinsäureamid (EP 0604 813) und Maleinsäureanhydrid sowie Ammoniak freisetzende Verbindungen.

In Chemical Abstracts, Vol. 84, No. 11, 15. March 1976, Columbus, Ohio, US; Abstract No. 70 582 wird die saure Hydrolyse von Cyanophycin zu Arginin mit 0,03 m Essigsäure bei 105°C beschrieben.

In der vorliegenden Erfindung nun werden biologische Methoden zur Herstellung von Asparaginsäure Homo- und Copolymeren.

Bislang wurden in der Natur drei unterschiedliche Polyaminosäuren Poly-γ-Glutamat, Poly-ε-Lysin und Poly-α-Arginylaspartat (Cyanophycin) gefunden.

Poly-γ-Glutamat wird von verschiedenen Gram-positiven Bakterien wie z.B. Bacillus licheniformis, Bacillus subtilis natto, oder Bacillus anthracis produziert. Poly-ε-Lysin wird von Streptomyces albulus hergestellt.

Poty-α-Arginylaspartat wird von vielen Cyanobakterien wie beispielsweise Spirulina platensis, Aphanocapsa PCC 630S oder Anabena cylindrica produziert. Die Synthese erfolgt auf einem non ribosomalem Weg, wobei ein Polypeptid mit polydisperser Molekulargewichtsverteilung erhalten und in Form von Cyanophycin-Granalien im Zellinneren gelagert wird.

Aus der Patentliteratur ist bislang nur die biotechnische Herstellung von Poly-γ-Glutamat unter Verwendung von Bacillus subtilis bzw. Bacillus licheniformis bekannt. (JP 1-174397 (1989), JP 43-24472 (1969) und US 2 895 882).

Wir haben nun gefunden, dass sich Asparaginsäure Homo- und Copolymere unter Abspaltung von Arginin unter basischen-Bedingungen aus Cyanophycin, das durch Fermentation mit Hilfe von Cyanobakterien hergestellt wird. Die erhaltenen Polymere besitzen folgende Strukturen.

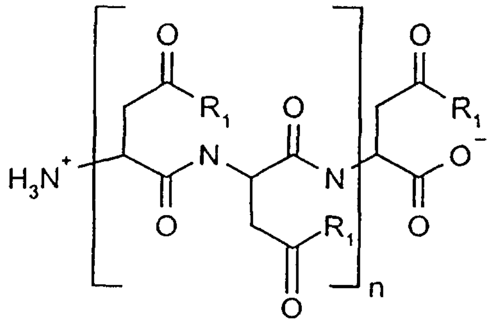

$R_1$ : = OH, oder Arginyl
n : 5-400

Wenn die Summe aller Reste $R_1$ 100% entspricht, so beträgt der Anteil $R_1$ = OH zwischen 5% und 100%, bevorzugt 30% -100% und besonders bevorzugt 70% bis 100%. Das Molekulargewicht der Polymere beträgt im allgemeinen zwischen 1 000 und 100 000 bevorzugt zwischen 2 000 und 50 000 besonders bevorzugt zwischen 2 000 und 30 000.

Die Summe n aller Wiederholungseinheiten ist abhängig von den Spaltungsbedingungen denen das Zwischenprodukt Cyanophycin unterworfen wird. Die Argininabspaltung kann sowohl sauer als auch basisch erfolgen. Wird die Hydrolyse sauer durchgeführt, sind in Relation zum eingebauten Arginin stöchiometrische Mengen Säure notwendig, da die Säure als Argininsalz abgefangen wird. Als Säure können alle Mineralsäuren wie z. B. Salzsäure, Schwefelsäuren, Phosphorsäuren und niedere Fettsäuren von $C_1$-$C_5$ eingesetzt werden. Weiterhin kann die hydrolytische Spaltung auch unter Druck unter Verwendung von Kohlensäure oder $CO_2$ erfolgen. In Abhängigkeit von der Konzentration der eingesetzten Säure und den Reaktionsbedingungen kann neben der Argininabspaltung auch eine Depolymerisation durch hydrolytische Spaltung der Polyaspartatkette stattfinden. Durch geeignete Wahl der Reaktionsbedingungen, wie verdünnte Säure, mittlere Reaktionszeiten, Temperaturen von max. 100°C, kann die nicht erwünschte Depolymerisation jedoch minimiert werden.

Gemäß der vorliegenden Erfindung wird die Hydrolyse jedoch unter basischen Bedingungen durchgeführt da unter diesen Bedingungen die Polyaspartatkette stabiler ist. Die Reaktion wird bei einem pH-Wert 9 - 12 und bei Temperaturen zwischen 20°C und 150°C bevorzugt 50°C -120°C durchgeführt Das Reaktionsprodukt wird nach der Hydrolyse durch Filtration vom nicht umgesetzten Cyanophycin und dem im Alkalischen unlöslichen Arginin getrennt. Als Base für die alkalische Hydrolyse eignen sich alle Metallhydroxide oder Carbonate, die im wässrigen Medium pH-Werte > 8,5 ermöglichen. Bevorzugt sind Alkali- und Erdalkalimetallhydroxide.

Das für die hydrolytische Bildung der Asparaginsäure Homo- und Copolymere eingesetzte Cyanophycin wird durch Fermentation aus Cyanobakterien wie beispielsweise Aphanocapsa PCC 6308, Anabena cylindrica oder Spirulina platensis gewonnen. Ein möglicher Biosyntheseweg wird im experimentellen Teil beschrieben.

Die als Produkte des beanspruchten Verfahrens erhaltenen Asparaginsäure Homo- und Copolymere wurden mit Elementaranalyse, Aminosäureanalytik und NMR-Spektroskopie charakterisiert. Das Molekulargewicht wurde mit Hilfe der wässrigen GPC bestimmt. Weiterhin wurden die Produkte anwendungstechnisch auf ihre Fähigkeit zur Inhibierung mineralischer Niederschläge wie Calciumcarbonat, Calciumsulfat, Calciumphosphat, Calciumoxalat und Bariumsulfat sowie auf ihr Dispergiervermögen für Feststoffpartikel geprüft. Das Calciumcarbonatinhibierungsvermögen wurde u.a. nach einer Vorschrift aus C.S.

Sikes, A.P. Wheeler in Chemical Aspects of Regulation of Mineralisation S. 53 - 57, University of South Alabama Publication Series (1988) durchgeführt. Die Produkte sind aufgrund ihrer natürlichen polypeptidischen Struktur, die auf $\alpha$-verknüpfter L-Asparaginsäure basiert, vollständig biologisch abbaubar.

Sie können beispielsweise als Cobuilder in Wasch- und Reinigungsmitteln, zur Inhibierung und Dispergierung von Niederschlägen in Kühl- und Heizkreisläufen zur Verminderung und Dispergierung von Ablagerungen sowie zur Korrosionsminderung und Gashydratinhibierung bei der Erdöl- und Erdgasförderung eingesetzt werden.

**Beispiel 1**

Kulturbedingungen, Extraktion und Reinigung von Cyanophycin:

Das Cyanobacterium Aphanocapsa PCC6308 wird im 10 1-Fermenter (batch-Kultur) mit 9 1 BGII Medium unter phototrophen Bedingungen (6 000 lux, 1/d cycle 12/12) bei 30°C inkubiert und mit Luft (200 ml/min) begast.

Bevor die Zellen die stationäre Phase erreichen (nach 14 Tagen mit einer Optischen Dichte $OD_{665}$ von ca. 1.6) wird dem Medium 10 mg/l L-Arginin und/oder 200 mg/l $NaNO_3$ und 5 mg/ml Chloramphenicol zugegeben, und danach die Zellsuspension für weitere 48 h bei reduziertem Licht (600 lux) und erniedrigter Temperatur (20°C) inkubiert. Die Zellen werden durch Zentrifugation bei 10 000xg geerntet und zweimal in destilliertem Wasser gewaschen. Das Zellpellet (ca. 25 g Feuchtgewicht, ca. 3 g Trockenmasse) wird in 100 ml einer wäßrigen Pufferlösung (pH 7.2) aufgenommen. Die Zellen werden durch Ultraschall-Behandlung aufgeschlossen und danach für 15 h bei 4°C gerührt. Durch eine Zentrifugation bei 30 000 x g wird das Rohcyanophycin pelletiert. Das Rohcyanophycin wird in 60 ml $H_2O$ resuspendiert. Der durch eine dreimalige fraktionierte Zentrifugation bei 1 000xg gewonnene Überstand (S 1000) wird einer Zentrifugation bei 30 000 xg unterworfen und das so erhaltene Pellet in 0,1 n HCl gelöst (Ausbeute: ca. 1000 mg natives Cyanophycin). Durch dreimalige Retitration in 1 N NaOH wird das in 0.1 N HCl gelöste native Cyanophycin abschließend gereinigt (Ausbeute: ca 150 mg Cyanophycin).

Isoliert wurde der Stamm Aphanocapsa (= Synechocystis) PCC6308 ursprünglich im Jahre 1949 und G. C. Gerloff aus einem See in Wisconsin (USA) und erstmalig beschrieben von Gerloff et al. 1950 (Gerloff, G. C., Fitzgerald, G. P. & Skoog, F. 1950. The isolation, purification and nutrient solution requirements of blue-green algae. In Proceedings of the Symposium on the Culturing of Algae, pp. 27-44. Dayton, Ohio, U.S.A.: Charles F. Kettering Foundation).

Der in der vorliegenden Anmeldung verwendete Stamm Synechocystis PCC 6308 wurde im Namen der Bayer AG, 51368 Leverkusen, am 19. Februar 1998 bei der DSMZ-Deutsche Sammlung von Mikroorganismen und Zellkulturen GmbH, Mascheroder Weg 16, D-38124 Braunschweig mit der Nummer DSM 12037 hinterlegt.

## Beispiel 2

Basische Hydrolyse von Cyanophycin

500 mg Cyanophycin aus Beispiel 1 werden 5 ml Wasser suspendiert. Man gibt 75 mg NaOH (100%) zu und erwärmt die Mischung unter Rühren für 12 h auf 90°C. Anschließend wird die Mischung auf Raumtemperatur abgekühlt und filtriert Als Rückstand bleibt eine Mischung von Arginin und nicht umgesetztem Cyanophycin zurück. Im Filtrat liegen die Natriumsalze der Asparaginsäure Homo- und Copolymere vor.

Bestimmung des Calciumcarbonatinhibierungsvermögens nach einer modifizierten NACE[1)]-Methode: TM 0374-90

Einsatzstoffe:

| | | |
|---|---|---|
| Lösung 1: | 12,15 g<br>3,68 g | Calciumchlorid-dihydrat p.a.<br>Magnesiumchlorid-hexahydrat p.a.<br>mit destilliertem Wasser auf 1000 ml Lösung aufgefüllt. |
| Lösung 2: | 7,36 g | Natriumhydrogencarbonat p.a.<br>mit destilliertem Wasser auf 1000 ml Lösung aufgefüllt. |

Die Lösungen 1. und 2. sind jeweils frisch anzusetzen und vor ihrer Verwendung mit $CO_2$ zu sättigen.

100 ml Lösung 1. werden mit 1,2,3,5,10 ppm Inhibitor (Aktivsubstanz) bezogen auf die gesamte Testmischung versetzt. Anschließend werden 100 ml der Lösung 2. zugesetzt.

Anschließend wird die Testmischung verschlossen durch Schütteln vermischt und in einem auf 70°C vorgeheizten Wasserbad 16 h gelagert. (Zum Vergleich wird eine Probe ohne Inhibitor Zusatz in der Testreihe mitgeführt.) Nach dieser Zeit werden alle Proben gleichzeitig aus dem Wasserbad entfernt und auf 30°C abgekühlt. Von allen Proben werden jeweils 5 ml entnommen, über ein 0,45 µm Filter filtriert und zur Stabilisierung mit Salzsäure angesäuert.

Die Bestimmung des Calciumgehaltes im Filtrat erfolgt durch Titration gegen Indikator oder durch Atomabsorptionsspektroskopie.

Das Inhibierungsvermögen wird wie folgt berechnet:

$$\frac{a-b}{c-b} * 100 = \% \text{ Inhibierung}$$

a: Calciummenge in der Probe

1) NACE: National Association of Corrosion engeneers

b: Calciummenge in der Blindprobe nach Temperung
c: Calciummenge in der Blindprobe vor Temperung

| Inhibitor [ppm] | Beispiel 2 | Inhibierung [%] Polyasparaginsäure chemische Synthese |
|---|---|---|
| 1 | 20 | 44 |
| 2 | 39 | 59 |
| 3 | 66 | 72 |
| 5 | 83 | 85 |
| 10 | 90 | 94 |

## Patentansprüche

1. Verfahren zur Herstellung von Asparaginsäure Homo- und Copolymeren aus Cyanophycin, das durch Fermentation mit Hilfe von Cyanobakterien hergestellt wird, woraus dann Arginin abgespalten wird, **dadurch gekennzeichnet, dass** die Hydrolyse unter alkalischen Bedingungen bei pH 9-12 durchgeführt wird.

2. Verfahren nach Anspruch 1 **dadurch gekennzeichnet, dass** das Edukt Cyanophycin durch Fermentation mit Hilfe der Cyanobakterien Aphanocapsa PCC6308, Anabaena cylindrica oder Spirulina platensis hergestellt wird.

## Claims

1. Process for producing aspartic acid homo- and copolymers from cyanophycin which is produced by fermentation with the aid of cyanobacteria and from which arginine is then eliminated, **characterized in that** the hydrolysis is carried out under alkaline conditions at pH 9-12.

2. Process according to Claim 1, **characterized in that** the precursor cyanophycin is produced by fermentation with the aid of the cyanobacteria Aphanocapsa PCC6308, Anabaena cylindrica or Spirulina platensis.

## Revendications

1. Procédé de préparation d'homopolymères et de copolymères de l'acide aspartique à partir de cyanophycine préparée par fermentation à l'aide de cyanobactéries, d'où est séparée ensuite l'arginine, **caractérisé en ce que** l'hydrolyse est menée dans des conditions alcalines à pH 9-12.

2. Procédé selon la revendication 1, **caractérisé en ce que** l'éduit cyanophycine est préparé par fermentation à l'aide des cyanobactéries Aphanocapsa PCC6308, Anabaena cylindrica ou Spirulina platensis.